# EUROPEAN PATENT SPECIFICATION

(11) **EP 1 838 420 B1**
(45) Date of publication and mention of the grant of the patent: **17.02.2021**
(21) Application number: 05816567.1
(22) Date of filing: 02.11.2005
(51) Int. Cl.: C01B 39/48, C01B 39/12, B01J 29/86, C07C 209/16, C07C 209/30, B01D 53/94, C07C 1/20

(54) **BORON-CONTAINING MOLECULAR SIEVE CHA**
BORHALTIGES MOLSIEB CHA
TAMIS MOLÉCULAIRE CHA CONTENANT DU BORE

(30) Priority: 30.11.2004 US 632007 P; 30.11.2004 US 632008 P; 30.11.2004 US 632022 P; 30.11.2004 US 632005 P; 30.11.2004 US 632006 P
(43) Date of publication of application: 03.10.2007
(73) Proprietor: Chevron U.S.A. Inc., San Ramon, CA 94583 (US)
(72) Inventor: YUEN, Lun-Teh, San Ramon, California 94583 (US); ZONES, Stacey, I., San Ramon, California 94583 (US)
(74) Representative: Haseltine Lake Kempner LLP
(86) International application number: PCT/US2005/039647
(87) International publication number: WO 2006/060101

(56) References cited:
- WO-A2-2005/063622
- WO-A2-2005/063623
- US-A1- 2003 069 449
- US-A1- 2003 089 227
- US-A1- 2003 176 751
- US-A1- 2003 176 751
- US-A1- 2005 054 885
- US-A1- 2005 197 520
- LUN-TEH YUEN ET AL: "Product selectivity in methanol to hydrocarbon conversion for isostructural compositions of AFI and CHA molecular sieves", MICROPOROUS MATERIALS, vol. 2, no. 2, 1 February 1994 (1994-02-01), pages 105-117, XP55008277, ISSN: 0927-6513, DOI: 10.1016/0927-6513(93)E0039-J
- ELANANY M ET AL: "Periodic density functional investigation of Bronsted acidity in isomorphously substituted chabazite and AlPO-34 molecular sieves", MICROPOROUS AND MESOPOROUS MATERIALS, ELSEVIER SCIENCE PUBLISHING, NEW YORK, US, vol. 71, no. 1-3, 17 June 2004 (2004-06-17), pages 51-56, XP004512002, ISSN: 1387-1811, DOI: 10.1016/J.MICROMESO.2004.03.018
- CHRISTOPHER W. JONES ET AL: "Synthesis of Hydrophobic Molecular Sieves by Hydrothermal Treatment with Acetic Acid", CHEMISTRY OF MATERIALS, vol. 13, no. 3, 1 March 2001 (2001-03-01), pages 1041-1050, XP55008770, ISSN: 0897-4756, DOI: 10.1021/cm0007961

## Description

### BACKGROUND

Chabazite, which has the crystal structure designated "CHA", is a natural zeolite with the approximate formula Ca₆Al₁₂Si₂₄O₇₂. Synthetic forms of chabazite are described in "Zeolite Molecular Sieves" by D. W. Breck, published in 1973 by John Wiley & Sons. The synthetic forms reported by Breck are: zeolite "K-G", described in J. Chem. Soc., p. 2822 (1956), Barrer et al.; zeolite D, described in British Patent No. 868,846 (1961); and zeolite R, described in U. S. Patent No. 3,030,181, issued April 17, 1962 to Milton et al. Chabazite is also discussed in "Atlas of Zeolite Structure Types" (1978) by W. H. Meier and D. H. Olson.

The K-G zeolite material reported in the J. Chem. Soc. Article by Barrer et al. is a potassium form having a silica:alumina mole ratio (referred to herein as "SAR") of 2.3:1 to 4.15:1. Zeolite D reported in British Patent No. 868,846 is a sodium-potassium form having a SAR of 4.5:1 to 4.9:1. Zeolite R reported in U. S. Patent No. 3,030,181 is a sodium form which has a SAR of 3.45:1 to 3.65:1.

Citation No. 93:66052y in Volume 93 (1980) of Chemical Abstracts concerns a Russian language article by Tsitsishrili et al. in Soobsch. Akad. Nauk. Gruz. SSR 1980, 97(3) 621-4. This article teaches that the presence of tetramethylammonium ions in a reaction mixture containing K₂O-Na₂O-SiO₂-Al₂O₃-H₂O promotes the crystallization of chabazite. The zeolite obtained by the crystallization procedure has a SAR of 4.23.

The molecular sieve designated SSZ-13, which has the CHA crystal structure, is disclosed in U. S. Patent No. 4,544,538, issued October 1, 1985 to Zones. SSZ-13 is prepared from nitrogen-containing cations derived from 1-adamantamine, 3-quinuclidinol and 2-exo-aminonorbornane. Zones discloses that the SSZ-13 of U. S. Patent No. 4,544,538 has a composition, as-synthesized and in the anhydrous state, in terms of mole ratios of oxides as follows:
(0.5 to 1.4)R₂O : (0 to 0.5)M₂O : W₂O₃ : (greater than 5)YO₂
wherein M is an alkali metal cation, W is selected from aluminum, gallium and mixtures thereof, Y is selected from silicon, germanium and mixtures thereof, and R is an organic cation. U. S. Patent No. 4,544,538 does not, however, disclose boron-containing SSZ-13.

U. S. Patent No. 6,709,644, issued March 23, 2004 to Zones et al., discloses zeolites having the CHA crystal structure and having small crystallite sizes. It does not, however, disclose a CHA zeolite containing boron. It is disclosed that the zeolite can be used for separation of gasses (e.g., separating carbon dioxide from natural gas), and in catalysts used for the reduction of oxides of nitrogen in a gas stream (e.g., automotive exhaust), converting lower alcohols and other oxygenated hydrocarbons to liquid products, and for producing dimethylamine. Lun-Teh Yuen et al., "Product selectivity in methanol to hydrocarbon conversion for isostructural conversions of AFI and CHA molecular sieves", Microporous materials, vol. 2, no. 2, 1 Feb 1994, pp 105-117, describes AFI and CHA molecular sieves. US 2003/0176751 describes a porous crystalline material having the chabazite framework type.

### SUMMARY OF THE INVENTION

In accordance with the present invention there is provided a boron-containing molecular sieve having the CHA crystal structure and comprising (1) silicon oxide and (2) a combination of boron oxide and aluminum oxide, and optionally iron oxide, titanium oxide, gallium oxide and mixtures thereof, wherein oxide (2) is more than 50% boron oxide on a molar basis.

In accordance with this invention there is provided a method for preparing a boron-containing molecular sieve having the CHA crystal structure and comprising (1) silicon oxide and (2) a combination of boron oxide and aluminum oxide, and optionally iron oxide, titanium oxide, gallium oxide and mixtures thereof, wherein oxide (2) is more than 50% boron oxide on a molar basis, said method comprising:
A. forming an aqueous reaction mixture consisting of a composition in terms of mole ratios falling within the following ranges:

| | |
|---|---|
| YO₂/WₐO_{b} | >2 - 2,000 |
| OH-/YO₂ | 0.2 - 0.45 |
| Q/YO₂ | 0.2 - 0.45 |
| M_{2/n}O/YO₂ | 0 - 0.25 |
| H₂O/YO₂ | 22 - 80 |

wherein Y is silicon, W is a combination of boron and aluminium, and optionally iron, titanium, gallium and mixtures thereof; a is 1 or 2 and b is 2 when a is 1 (i.e., W is tetravalent) or b is 3 when a is 2 (i.e., W is trivalent); M is an alkali metal or alkaline earth metal, n is the valence of M (i.e., 1 or 2), and Q is a quaternary ammonium cation derived from 1-adamantamine, 3-quinuclidinol or 2-exo-aminonorbornane; and wherein Q is associated with an anion, A⁻, which is not detrimental to the formation of the molecular sieve, and wherein A⁻ is hydroxide; and
B. maintaining said aqueous mixture under sufficient crystallization conditions until crystals are formed.

In accordance with this invention, there is also provided a boron-containing molecular sieve as set out in claim 1 and having a composition, as-synthesized and in the anhydrous state, in terms of mole ratios of oxides as follows:

| | |
|---|---|
| YO₂/W_{c}O_{d} | 20 - 2,000 |
| M_{2/n}O/YO₂ | 0 - 0.03 |
| Q/YO₂ | 0.02 - 0.05 |

wherein Y is silicon, W is a combination of boron and aluminium, and optionally iron, titanium, gallium and mixtures thereof; c is 1 or 2; d is 2 when c is 1 (i.e., W is tetravalent) or d is 3 or 5 when c is 2 (i.e., d is 3 when W is trivalent or 5 when W is pentavalent); M is an alkali metal cation, alkaline earth metal cation or mixtures thereof; n is the valence of M (i.e., 1 or 2); and Q is a quaternary ammonium cation derived from 1-adamantamine, 3-quinuclidinol or 2-exo-aminonorbornane.

The present invention relates to a process for the production of light olefins comprising olefins having from 2 to 4 carbon atoms per molecule from an oxygenate feedstock. The process comprises passing the oxygenate feedstock to an oxygenate conversion zone containing a molecular sieve catalyst to produce a light olefin stream.

Thus, in accordance with the present invention there is provided a process for the production of light olefins from a feedstock comprising an oxygenate or mixture of oxygenates, the process comprising reacting the feedstock at effective conditions over a catalyst comprising boron-containing molecular sieve as set out in claim 1.

In accordance with the present invention there is provided a process for producing methylamine or dimethylamine comprising reacting methanol, dimethyl ether or a mixture thereof and ammonia in the gaseous phase in the presence of a catalyst comprising a boron-containing molecular sieve as set out in claim 1.

In accordance with this invention, there is provided a process for the reduction of oxides of nitrogen contained in a gas stream wherein said process comprises contacting the gas stream with a molecular sieve, the molecular sieve having the CHA crystal structure and comprising (1) silicon oxide, and (2) a combination of boron oxide and aluminum oxide, and optionally iron oxide, titanium oxide, gallium oxide and mixtures thereof, wherein oxide (2) is more than 50% boron oxide on a molar basis. The molecular sieve may contain a metal or metal ions (such as cobalt, copper, platinum, iron, chromium, manganese, nickel, zinc, lanthanum, palladium, rhodium or mixtures thereof) capable of catalyzing the reduction of the oxides of nitrogen, and the process may be conducted in the presence of a stoichiometric excess of oxygen. In a preferred embodiment, the gas stream is the exhaust stream of an internal combustion engine.

In accordance with the present invention there is provided a process for separating gasses using a membrane containing a molecular sieve, the process comprising using as the molecular sieve a boron-containing molecular sieve having the CHA crystal structure and comprising (1) silicon oxide, and (2) a combination of boron oxide and aluminum oxide, and optionally iron oxide, titanium oxide, gallium oxide and mixtures thereof, wherein oxide (2) is more than 50% boron oxide on a molar basis.

This invention also relates to a process for treating an engine exhaust stream and in particular to a process for minimizing emissions during the cold start operation of an engine. Accordingly, the present invention provides a process for treating a cold-start engine exhaust gas stream containing hydrocarbons and other pollutants consisting of flowing said engine exhaust gas stream over a molecular sieve bed which preferentially adsorbs the hydrocarbons over water to provide a first exhaust stream, and flowing the first exhaust gas stream over a catalyst to convert any residual hydrocarbons and other pollutants contained in the first exhaust gas stream to innocuous products and provide a treated exhaust stream and discharging the treated exhaust stream into the atmosphere, the molecular sieve bed characterized in that it comprises a boron-containing molecular sieve having the CHA crystal structure and comprising (1) silicon oxide, and (2) a combination of boron oxide and aluminum oxide, and optionally iron oxide, titanium oxide gallium oxide and mixtures thereof, wherein oxide (2) is more than 50% boron oxide on a molar basis.

The present invention further provides such a process wherein the engine is an internal combustion engine, including automobile engines, which can be fueled by a hydrocarbonaceous fuel.

Also provided by the present invention is such a process wherein the molecular sieve has deposited on it a metal selected from the group consisting of platinum, palladium, rhodium, ruthenium, and mixtures thereof.

### DETAILED DESCRIPTION

The present invention relates to molecular sieves having the CHA crystal structure as set out in claim 1.

Boron-containing CHA molecular sieves can be suitably prepared from an aqueous reaction mixture containing sources of sources of an oxide of silicon; sources of boron oxide or a combination of boron oxide and aluminum oxide, iron oxide, titanium oxide, gallium oxide and mixtures thereof; optionally sources of an alkali metal or alkaline earth metal oxide; and a cation derived from 1-adamantamine, 3-quinuclidinol or 2-exo-aminonorbornane. The mixture should have a composition in terms of mole ratios falling within the ranges shown in Table A below:

**TABLE A**

| | |
|---|---|
| YO₂/WₐO_{b} | >2 - 2,000 |
| OH-/YO₂ | 0.2 - 0.45 |
| Q/YO₂ | 0.2 - 0.45 |
| M_{2/n}O/YO₂ | 0 - 0.25 |
| H₂O/YO₂ | 22 - 80 |

wherein Y is silicon; W is a combination of boron and aluminum, and optionally iron, titanium, gallium and mixtures thereof; M is an alkali metal or alkaline earth metal; n is the valence of M (i.e., 1 or 2) and Q is a quaternary ammonium cation derived from 1-adamantamine, 3-quinuclidinol or 2-exo-aminonorbornane (commonly known as a structure directing agent or "SDA").

The quaternary ammonium cation derived from 1-adamantamine can be a N,N,N-trialkyl-1-adamantammonium cation which has the formula: 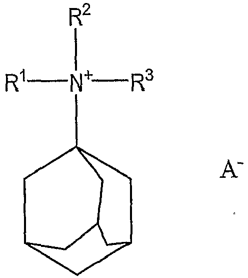 where R¹, R², and R³ are each independently a lower alkyl, for example methyl. The cation is associated with an anion, A⁻, which is not detrimental to the formation of the molecular sieve.

A⁻ is hydroxide. Hydroxide is the preferred anion. It may be beneficial to ion exchange, for example, a halide for hydroxide ion, thereby reducing or eliminating the alkali metal or alkaline earth metal hydroxide required.

The quaternary ammonium cation may be derived from 3-quinuclidinol.

The quaternary ammonium cation derived from 2-exo-aminonorbornane can have the formula: 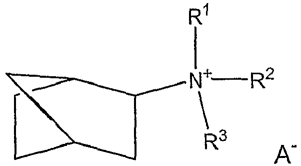 where R¹, R², R³ and A are as defined above.

The reaction mixture is prepared using standard molecular sieve preparation techniques. Typical sources of silicon oxide include fumed silica, silicates, silica hydrogel, silicic acid, colloidal silica, tetra-alkyl orthosilicates, and silica hydroxides. Sources of boron oxide include borosilicate glasses and other reactive boron compounds. These include borates, boric acid and borate esters. Typical sources of aluminum oxide include aluminates, alumina, hydrated aluminum hydroxides, and aluminum compounds such as AlCl₃ and Al₂(SO₄)₃. Sources of other oxides are analogous to those for silicon oxide, boron oxide and aluminum oxide.

It has been found that seeding the reaction mixture with CHA crystals both directs and accelerates the crystallization, as well as minimizing the formation of undesired contaminants. In order to produce pure phase boron-containing CHA crystals, seeding may be required. When seeds are used, they can be used in an amount that is about 2-3 weight percent based on the weight of YO₂.

The reaction mixture is maintained at an elevated temperature until CHA crystals are formed. The temperatures during the hydrothermal crystallization step are typically maintained from about 120°C to about 160°C. It has been found that a temperature below 160°C, e.g., about 120°C to about 140°C, is useful for producing boron-containing CHA crystals without the formation of secondary crystal phases.

The crystallization period is typically greater than 1 day and preferably from about 3 days to about 7 days. The hydrothermal crystallization is conducted under pressure and usually in an autoclave so that the reaction mixture is subject to autogenous pressure. The reaction mixture can be stirred, such as by rotating the reaction vessel, during crystallization.

Once the boron-containing CHA crystals have formed, the solid product is separated from the reaction mixture by standard mechanical separation techniques such as filtration. The crystals are water-washed and then dried, e.g., at 90°C to 150°C for from 8 to 24 hours, to obtain the as-synthesized crystals. The drying step can be performed at atmospheric or subatmospheric pressures.

The boron-containing CHA molecular sieve has a composition, as-synthesized and in the anhydrous state, in terms of mole ratios of oxides as indicated in Table B below:
As-Synthesized Boron-containing CHA Composition

**TABLE B**

| | |
|---|---|
| YO₂/W_{c}O_{d} | 20 - 2,000 |
| M_{2/n}O/YO₂ | 0 - 0.03 |
| Q/YO₂ | 0.02 - 0.05 |

where Y, W, M, n and Q are as defined above.

The boron-containing CHA molecular sieves, as-synthesized, have a crystalline structure whose X-ray powder diffraction ("XRD") pattern shows the following characteristic lines:

**TABLE I**

| As-Synthesized Boron-Containing CHA XRD | | |
|---|---|---|
| 2 Theta^{(a)} | d-spacing (Angstroms) | Relative Intensity^{(b)} |
| 9.68 | 9.13 | S |
| 14.17 | 6.25 | M |
| 16.41 | 5.40 | VS |
| 17.94 | 4.94 | M |
| 21.13 | 4.20 | VS |
| 25.21 | 3.53 | VS |
| 26.61 | 3.35 | W-M |
| 31.11 | 2.87 | M |
| 31.42 | 2.84 | M |
| 31.59 | 2.83 | M |

| | | |
|---|---|---|
| ^{(a)} ± 0.10 ^{(b)} The X-ray patterns provided are based on a relative intensity scale in which the strongest line in the X-ray pattern is assigned a value of 100: W(weak) is less than 20; M(medium) is between 20 and 40; S(strong) is between 40 and 60; VS(very strong) is greater than 60. | | |

Table IA below shows the X-ray powder diffraction lines for as-synthesized boron-containing CHA including actual relative intensities.

**TABLE IA**

| As-Synthesized Boron-Containing CHA XRD | | |
|---|---|---|
| 2 Theta^{(a)} | d-spacing (Angstroms) | Relative Intensity (%) |
| 9.68 | 9.13 | 55.2 |
| 13.21 | 6.70 | 5.4 |
| 14.17 | 6.25 | 33.5 |
| 16.41 | 5.40 | 81.3 |
| 17.94 | 4.94 | 32.6 |
| 19.43 | 4.56 | 6.8 |
| 21.13 | 4.20 | 100 |
| 22.35 | 3.97 | 15.8 |
| 23.00 | 3.86 | 10.1 |
| 23.57 | 3.77 | 5.1 |
| 25.21 | 3.53 | 78.4 |
| 26.61 | 3.35 | 20.2 |
| 28.37 | 3.14 | 6.0 |
| 28.57 | 3.12 | 4.4 |
| 30.27 | 2.95 | 3.9 |
| 31.11 | 2.87 | 29.8 |
| 31.42 | 2.84 | 38.3 |
| 31.59 | 2.83 | 26.5 |
| 32.27 | 2.77 | 1.4 |
| 33.15 | 2.70 | 3.0 |
| 33.93 | 2.64 | 4.7 |
| 35.44 | 2.53 | 3.9 |
| 35.84 | 2.50 | 1.2 |
| 36.55 | 2.46 | 10.9 |
| 39.40 | 2.29 | 1.8 |
| 40.02 | 2.25 | 1.3 |
| 40.44 | 2.23 | 1.0 |
| 40.73 | 2.21 | 6.0 |

| | | |
|---|---|---|
| ^{(a)} ± 0.10 | | |

After calcination, the boron-containing CHA molecular sieves have a crystalline structure whose X-ray powder diffraction pattern include the characteristic lines shown in Table II:

**TABLE II**

| Calcined Boron-Containing CHA XRD | | |
|---|---|---|
| 2 Theta^{(a)} | d-spacinq (Angstroms) | Relative Intensity |
| 9.74 | 9.07 | VS |
| 13.12 | 6.74 | M |
| 14.47 | 6.12 | W |
| 16.38 | 5.41 | W |
| 18.85 | 4.78 | M |
| 21.07 | 4.21 | M |
| 25.98 | 3.43 | W |
| 26.46 | 3.37 | W |
| 31.30 | 2.86 | W |
| 32.15 | 2.78 | W |

| | | |
|---|---|---|
| ^{(a)} ± 0.10 | | |

Table IIA below shows the X-ray powder diffraction lines for calcined boron-containing CHA including actual relative intensities.

**TABLE IIA**

| Calcined Boron-Containing CHA XRD | | |
|---|---|---|
| 2 Theta^{(a)} | d-spacing (Angstroms) | Relative Intensity (%) |
| 9.74 | 9.07 | 100 |
| 13.12 | 6.74 | 29.5 |
| 14.47 | 6.12 | 4.6 |
| 16.38 | 5.41 | 14.2 |
| 18.85 | 4.78 | 22.1 |
| 19.60 | 4.53 | 2.2 |
| 21.07 | 4.21 | 32.9 |
| 22.84 | 3.89 | 2.2 |
| 23.68 | 3.75 | 0.8 |
| 25.98 | 3.43 | 13.1 |
| 26.46 | 3.37 | 8.7 |
| 28.27 | 3.15 | 1.3 |
| 29.24 | 3.05 | 1.6 |
| 30.32 | 2.95 | 1.7 |
| 31.30 | 2.86 | 14.4 |
| 32.15 | 2.78 | 9.0 |
| 32.56 | 2.75 | 0.2 |
| 35.26 | 2.54 | 2.4 |

| | | |
|---|---|---|
| ^{(a)} ± 0.10 | | |

The X-ray powder diffraction patterns were determined by standard techniques. The radiation was the K-alpha/doublet of copper and a scintillation counter spectrometer with a strip-chart pen recorder was used. The peak heights I and the positions, as a function of 2 Theta where Theta is the Bragg angle, were read from the spectrometer chart. From these measured values, the relative intensities, 100 x I/Io, where Io is the intensity of the strongest line or peak, and d, the interplanar spacing in Angstroms corresponding to the recorded lines, can be calculated.

Variations in the diffraction pattern can result from variations in the mole ratio of oxides from sample to sample. The molecular sieve produced by exchanging the metal or other cations present in the molecular sieve with various other cations yields a similar diffraction pattern, although there can be shifts in interplanar spacing as well as variations in relative intensity. Calcination can also cause shifts in the X-ray diffraction pattern. Also, the symmetry can change based on the relative amounts of boron and aluminum in the crystal structure. Notwithstanding these perturbations, the basic crystal lattice structure remains unchanged.

Boron-containing CHA molecular sieves are useful in adsorption, in catalysts useful in converting methanol to olefins, synthesis of amines (such as dimethylamine), in the reduction of oxides of nitrogen in gasses (such as automobile exhaust), and in gas separation.

The present invention comprises a process for catalytic conversion of a feedstock comprising one or more oxygenates comprising alcohols and ethers to a hydrocarbon product containing light olefins, i.e., C₂, C₃ and/or C₄ olefins. The feedstock is contacted with the molecular sieve of the present invention at effective process conditions to produce light olefins.

The term "oxygenate" as used herein designates compounds such as alcohols, ethers and mixtures thereof. Examples of oxygenates include, but are not limited to, methanol and dimethyl ether.

The process of the present invention may be conducted in the presence of one or more diluents which may be present in the oxygenate feed in an amount between about 1 and about 99 molar percent, based on the total number of moles of all feed and diluent components. Diluents include, but are not limited to, helium, argon, nitrogen, carbon monoxide, carbon dioxide, hydrogen, water, paraffins, hydrocarbons (such as methane and the like), aromatic compounds, or mixtures thereof. U. S. Patents No. 4,861,938 and 4,677,242
emphasize the use of a diluent to maintain catalyst selectivity toward the production of light olefins, particularly ethylene.

The oxygenate conversion is preferably conducted in the vapor phase such that the oxygenate feedstock is contacted in a vapor phase in a reaction zone with the molecular sieve of this invention at effective process conditions to produce hydrocarbons, i.e., an effective temperature, pressure, weight hourly space velocity (WHSV) and, optionally, an effective amount of diluent. The process is conducted for a period of time sufficient to produce the desired light olefins. In general, the residence time employed to produce the desired product can vary from seconds to a number of hours. It will be readily appreciated that the residence time will be determined to a significant extent by the reaction temperature , the molecular sieve catalyst, the WHSV, the phase (liquid or vapor) and process design characteristics. The oxygenate feedstock flow rate affects olefin production. Increasing the feedstock flow rate increases WHSV and enhances the formation of olefin production relative to paraffin production. However, the enhanced olefin production relative to paraffin production is offset by a diminished conversion of oxygenate to hydrocarbons.

The oxygenate conversion process is effectively carried out over a wide range of pressures, including autogenous pressures. At pressures between about 0.01 atmospheres (0.1 kPa) and about 1000 atmospheres (101.3 kPa), the formation of light olefins will be affected although the optimum amount of product will not necessarily be formed at all pressures. The preferred pressure is between about 0.01 atmospheres (0.1 kPa) and about 100 atmospheres (10.13 kPa). More preferably, the pressure will range from about 1 to about 10 atmospheres (101.3 kPa to 1.013 Mpa). The pressures referred to herein are exclusive of the diluent, if any, that is present and refer to the partial pressure of the feedstock as it relates to oxygenate compounds.

The temperature which may be employed in the oxygenate conversion process may vary over a wide range depending, at least in part, on the molecular sieve catalyst. In general, the process can be conducted at an effective temperature between about 200°C and about 700°C. At the lower end of the temperature range, and thus generally at a lower rate of reaction, the formation of the desired light olefins may become low. At the upper end of the range , the process may not form an optimum amount of light olefins and catalyst deactivation may be rapid.

The molecular sieve catalyst preferably is incorporated into solid particles in which the catalyst is present in an amount effective to promote the desired conversion of oxygenates to light olefins. In one aspect, the solid particles comprise a catalytically effective amount of the catalyst and at least one matrix material selected from the group consisting of binder materials, filler materials and mixtures thereof to provide a desired property or properties, e.g., desired catalyst dilution, mechanical strength and the like to the solid particles. Such matrix materials are often, to some extent, porous in nature and may or may not be effective to promote the desired reaction. Filler and binder materials include, for example, synthetic and naturally occurring substances such as metal oxides, clays, silicas, aluminas, silica-aluminas, silica-magnesias, silica-zirconias, silica-thorias and the like. If matrix materials are included in the catalyst composition, the molecular sieve preferably comprises about 1 to 99%, more preferably about 5 to 90%, and still more preferably about 10 to 80% by weight of the total composition.

The molecular sieve of the present invention can be used in a catalyst to prepare methylamine or dimethylamine. Dimethylamine is generally prepared in industrial quantities by continuous reaction of methanol (and/or dimethylether) and ammonia in the presence of a silica-alumina catalyst. The reactants are typically combined in the vapor phase, at temperatures in the range of 300°C to 500°C, and at elevated pressures. Such a process is disclosed in U. S. Patent No. 4,737,592, issued April 12, 1988 to Abrams et al.

The catalyst is used in its acid form. Acid forms of molecular sieves can be prepared by a variety of techniques. Preferably, the molecular sieve used to prepare dimethylamine will be in the hydrogen form, or have an alkali or alkaline earth metal, such as Na, K, Rb, or Cs, ion-exchanged into it.

The process of the present invention may involve reacting methanol, dimethylether or a mixture thereof and ammonia in amounts sufficient to provide a carbon/nitrogen (C/N) ratio from about 0.2 to about 1.5, preferably about 0.5 to about 1.2. The reaction is conducted at a temperature from about 250°C to about 450°C, preferably about 300°C to about 400°C. Reaction pressures can vary from about 7-7000 kPa (1-1000 psi), preferably about 70-3000 kPa (10-500 psi). A methanol and/or dimethylether space time of about 0.01-80 hours, preferably 0.10-1.5 hours, is typically used. This space time is calculated as the mass of catalyst divided by the mass flow rate of methanol/dimethylether introduced into the reactor.

Boron-containing CHA molecular sieves may be used for the catalytic reduction of the oxides of nitrogen in a gas stream. Typically, the gas stream also contains oxygen, often, a stoichiometric excess thereof. Also, the molecular sieve may contain a metal or metal ions within or on it which are capable of catalyzing the reduction of the nitrogen oxides. Examples of such metals or metal ions include cobalt, copper, platinum, iron, chromium, manganese, nickel, zinc, lanthanum, palladium, rhodium and mixtures thereof.

One example of such a process for the catalytic reduction of oxides of nitrogen in the presence of a zeolite is disclosed in U.S. Patent No. 4,297,328, issued October 27, 1981 to Ritscher et al.

There, the catalytic process is the combustion of carbon monoxide and hydrocarbons and the catalytic reduction of the oxides of nitrogen contained in a gas stream, such as the exhaust gas from an internal combustion engine. The zeolite used is metal ion-exchanged, doped or loaded sufficiently so as to provide an effective amount of catalytic copper metal or copper ions within or on the zeolite. In addition, the process is conducted in an excess of oxidant, e.g., oxygen.

The molecular sieve of the present invention can be used to separate gasses. For example, it can be used to separate carbon dioxide from natural gas. Typically, the molecular sieve is used as a component in a membrane that is used to separate the gasses. Examples of such membranes are disclosed in U.S. Patent No. 6,508,860, issued January 21, 2003 to Kulkarni et al.

The present invention relates to a process for treating engine exhaust using a boron-containing molecular sieve having the CHA crystal structure and comprising (1) silicon oxide, and (2) a combination of boron oxide and aluminum oxide, and optionally iron oxide, titanium oxide, gallium oxide and mixtures thereof, wherein oxide (2) is more than 50% boron oxide on a molar basis.

As stated this invention generally relates to a process for treating an engine exhaust stream and in particular to a process for minimizing emissions during the cold start operation of an engine. The engine consists of any internal or external combustion engine which generates an exhaust gas stream containing noxious components or pollutants including unburned or thermally degraded hydrocarbons or similar organics. Other noxious components usually present in the exhaust gas include nitrogen oxides and carbon monoxide. The engine may be fueled by a hydrocarbonaceous fuel. As used in this specification and in the appended claims, the term "hydrocarbonaceous fuel" includes hydrocarbons, alcohols and mixtures thereof. Examples of hydrocarbons which can be used to fuel the engine are the mixtures of hydrocarbons which make up gasoline or diesel fuel. The alcohols which may be used to fuel engines include ethanol and methanol. Mixtures of alcohols and mixtures of alcohols and hydrocarbons can also be used. The engine may be a jet engine, gas turbine, internal combustion engine, such as an automobile, truck or bus engine, a diesel engine or the like. The process of this invention is particularly suited for hydrocarbon, alcohol, or hydrocarbon-alcohol mixture, internal combustion engine mounted in an automobile. For convenience the description will use hydrocarbon as the fuel to exemplify the invention.

The use of hydrocarbon in the subsequent description is not to be construed as limiting the invention to hydrocarbon fueled engines.

When the engine is started up, it produces a relatively high concentration of hydrocarbons in the engine exhaust gas stream as well as other pollutants. Pollutants will be used herein to collectively refer to any unburned fuel components and combustion byproducts found in the exhaust stream. For example, when the fuel is a hydrocarbon fuel, hydrocarbons, nitrogen oxides, carbon monoxide and other combustion byproducts will be found in the engine exhaust gas stream. The temperature of this engine exhaust stream is relatively cool, generally below 500° C. and typically in the range of 200° to 400° C. This engine exhaust stream has the above characteristics during the initial period of engine operation, typically for the first 30 to 120 seconds after startup of a cold engine. The engine exhaust stream will typically contain, by volume, about 500 to 1000 ppm hydrocarbons.

The engine exhaust gas stream which is to be treated is flowed over a molecular sieve bed comprising the molecular sieve of this invention to produce a first exhaust stream. The molecular sieve is described below. The first exhaust stream which is discharged from the molecular sieve bed is now flowed over a catalyst to convert the pollutants contained in the first exhaust stream to innocuous components and provide a treated exhaust stream which is discharged into the atmosphere. It is understood that prior to discharge into the atmosphere, the treated exhaust stream may be flowed through a muffler or other sound reduction apparatus well known in the art.

The catalyst which is used to convert the pollutants to innocuous components is usually referred to in the art as a three-component control catalyst because it can simultaneously oxidize any residual hydrocarbons present in the first exhaust stream to carbon dioxide and water, oxidize any residual carbon monoxide to carbon dioxide and reduce any residual nitric oxide to nitrogen and oxygen. In some cases the catalyst may not be required to convert nitric oxide to nitrogen and oxygen, e.g., when an alcohol is used as the fuel. In this case the catalyst is called an oxidation catalyst. Because of the relatively low temperature of the engine exhaust stream and the first exhaust stream, this catalyst does not function at a very high efficiency, thereby necessitating the molecular sieve bed.

When the molecular sieve bed reaches a sufficient temperature, typically about 150-200° C., the pollutants which are adsorbed in the bed begin to desorb and are carried by the first exhaust stream over the catalyst. At this point the catalyst has reached its operating temperature and is therefore capable of fully converting the pollutants to innocuous components.

The adsorbent bed used in the instant invention can be conveniently employed in particulate form or the adsorbent can be deposited onto a solid monolithic carrier. When particulate form is desired, the adsorbent can be formed into shapes such as pills, pellets, granules, rings, spheres, etc. In the employment of a monolithic form, it is usually most convenient to employ the adsorbent as a thin film or coating deposited on an inert carrier material which provides the structural support for the adsorbent. The inert carrier material can be any refractory material such as ceramic or metallic materials. It is desirable that the carrier material be unreactive with the adsorbent and not be degraded by the gas to which it is exposed. Examples of suitable ceramic materials include sillimanite, petalite, cordierite, mullite, zircon, zircon mullite, spondumene, alumina-titanate, etc. Additionally, metallic materials which are within the scope of this invention include metals and alloys as disclosed in U.S. Pat. No. 3,920,583 which are oxidation resistant and are otherwise capable of withstanding high temperatures.

The carrier material can best be utilized in any rigid unitary configuration which provides a plurality of pores or channels extending in the direction of gas flow. It is preferred that the configuration be a honeycomb configuration. The honeycomb structure can be used advantageously in either unitary form, or as an arrangement of multiple modules. The honeycomb structure is usually oriented such that gas flow is generally in the same direction as the cells or channels of the honeycomb structure. For a more detailed discussion of monolithic structures, refer to U.S. Pat. Nos. 3,785,998 and 3,767,453.

The molecular sieve is deposited onto the carrier by any convenient way well known in the art. A preferred method involves preparing a slurry using the molecular sieve and coating the monolithic honeycomb carrier with the slurry. The slurry can be prepared by means known in the art such as combining the appropriate amount of the molecular sieve and a binder with water. This mixture is then blended by using means such as sonification, milling, etc. This slurry is used to coat a monolithic honeycomb by dipping the honeycomb into the slurry, removing the excess slurry by draining or blowing out the channels, and heating to about 100° C. If the desired loading of molecular sieve is not achieved, the above process may be repeated as many times as required to achieve the desired loading.

Instead of depositing the molecular sieve onto a monolithic honeycomb structure, one can take the molecular sieve and form it into a monolithic honeycomb structure by means known in the art.

The adsorbent may optionally contain one or more catalytic metals dispersed thereon. The metals which can be dispersed on the adsorbent are the noble metals which consist of platinum, palladium, rhodium, ruthenium, and mixtures thereof. The desired noble metal may be deposited onto the adsorbent, which acts as a support, in any suitable manner well known in the art. One example of a method of dispersing the noble metal onto the adsorbent support involves impregnating the adsorbent support with an aqueous solution of a decomposable compound of the desired noble metal or metals, drying the adsorbent which has the noble metal compound dispersed on it and then calcining in air at a temperature of about 400° to about 500° C. for a time of about 1 to about 4 hours. By decomposable compound is meant a compound which upon heating in air gives the metal or metal oxide.

Examples of the decomposable compounds which can be used are set forth in U.S. Pat. No. 4,791,091. Preferred decomposable compounds are chloroplatinic acid, rhodium trichloride, chloropalladic acid, hexachloroiridate (IV) acid and hexachlororuthenate. It is preferable that the noble metal be present in an amount ranging from about 0.01 to about 4 weight percent of the adsorbent support. Specifically, in the case of platinum and palladium the range is 0.1 to 4 weight percent, while in the case of rhodium and ruthenium the range is from about 0.01 to 2 weight percent.

These catalytic metals are capable of oxidizing the hydrocarbon and carbon monoxide and reducing the nitric oxide components to innocuous products. Accordingly, the adsorbent bed can act both as an adsorbent and as a catalyst.

The catalyst which is used in this invention is selected from any three component control or oxidation catalyst well known in the art. Examples of catalysts are those described in U.S. Pat. Nos. 4,528,279; 4,791,091; 4,760,044; 4,868,148; and 4,868,149.

Preferred catalysts well known in the art are those that contain platinum and rhodium and optionally palladium, while oxidation catalysts usually do not contain rhodium. Oxidation catalysts usually contain platinum and/or palladium metal. These catalysts may also contain promoters and stabilizers such as barium, cerium, lanthanum, nickel, and iron. The noble metals promoters and stabilizers are usually deposited on a support such as alumina, silica, titania, zirconia, aluminosilicates, and mixtures thereof with alumina being preferred. The catalyst can be conveniently employed in particulate form or the catalytic composite can be deposited on a solid monolithic carrier with a monolithic carrier being preferred. The particulate form and monolithic form of the catalyst are prepared as described for the adsorbent above.

### EXAMPLES

### Examples 1-14

Boron-containing CHA is synthesized by preparing the gel compositions, i.e., reaction mixtures, having the compositions, in terms of mole ratios, shown in the table below. The resulting gel is placed in a Parr bomb reactor and heated in an oven at the temperature indicated below while rotating at the speed indicated below. Products are analyzed by X-ray diffraction (XRD) and found to be boron-containing molecular sieves having the CHA structure. The source of silicon oxide is Cabosil M-5 fumed silica or HiSil 233 amorphous silica (0.208 wt.% alumina). The source of boron oxide is boric acid and the source of aluminum oxide is Reheis F 2000 alumina.

| Ex. # | SiO₂/B₂O₃ | SiO₂/Al₂O₃ | H₂O/SiO₂ | OH-/SiO₂ | Na+/SiO₂ | SDA/SiO₂) | Rx Cond.¹ | Seeds | %1-ada² |
|---|---|---|---|---|---|---|---|---|---|
| 1 | 2.51 | 1,010 | 23.51 | 0.25 | 0.20 | 0.25 | 140/43/5d | yes | 100 |
| 2 | 12.01 | 1,010 | 22.74 | 0.25 | 0.08 | 0.25 | 140/43/5d | yes | 100 |
| 3 | 12.33 | 1,010 | 22.51 | 0.25 | 0.08 | 0.25 | 140/43/5d | yes | 100 |
| 4 | 12.07 | 288,900 | 23.00 | 0.26 | 0.09 | 0.26 | 140/43/5d | no | 100 |
| 5 | 12.33 | 37,129 | 22.51 | 0.25 | 0.09 | 0.25 | 140/43/5d | yes | 100 |
| 6 | 12.33 | 248,388 | 22.51 | 0.25 | 0.09 | 0.25 | 140/43/5d | yes | 100 |
| 7 | 12.33 | 248,388 | 22.53 | 0.25 | 0.09 | 0.25 | 140/43/5d | yes | 100 |
| 8 | 12.33 | 248,388 | 22.53 | 0.25 | 0.00 | 0.25 | 140/43/5d | yes | 100 |
| 9 | 12.33 | 248,388 | 22.51 | 0.25 | 0.09 | 0.25 | 160/43/4d | yes | 100 |
| 10 | 11.99 | 288,900 | 23.18 | 0.26 | 0.09 | 0.26 | 160/43/4d | no | 100 |
| 11 | 12.13 | 288,900 | 32.22 | 0.43 | 0.21 | 0.21 | 160/43/4d | no | 100 |
| 12 | 11.99 | 288,900 | 23.16 | 0.26 | 0.00 | 0.26 | 160/43/4d | no | 100 |
| 13 | 11.99 | 288,900 | 23.18 | 0.26 | 0.09 | 0.26 | 160/43/4d | no | 100 |
| 14 | 3.08 | 248,388 | 22.51 | 0.25 | 0.00 | 0.25 | 140/43/6d | yes | 100 |

| | | | | | | | | | |
|---|---|---|---|---|---|---|---|---|---|
| ¹ °C/RPM/Days ² 1-ada = Quaternary ammonium cation derived from 1-adamantamine | | | | | | | | | |

### Examples 15-20

### Deboronation

Boron is removed from samples of the molecular sieves prepared as described in Example 13 above and then calcined. The sample is heated in an acid solution under the conditions indicated in the table below. The results are shown in the table.

| | Starting | | | Deboronation Rx | | | |
|---|---|---|---|---|---|---|---|
| Ex. No. | (B) SSZ-13 | 15 | 16 | 17 | 18 | 19 | 20 |
| Acid used | - | Acetic acid | acetic acid | acetic acid | HCl | HCl | HCl |
| Acid Molarity | - | 1.0 M | 0.01 M | 0.0001 M | 0.01 M | 0.001 M | 0.0001 M |
| Rx Cond. | - | 45C/Orpm/19h r | 45C/0rpm/19h r | 45C/0rpm/19h r | 45C/Orpm/19h r | 45C/0rpm/19h r | 45C/Orpm/19h r |

| | Untreated | Treated | Treated | Treated | Treated | Treated | Treated |
|---|---|---|---|---|---|---|---|
| Analysis Results | | | | | | | |
| Boron | 0.66% | 614ppm | 513ppm | 420ppm | 421 ppm | 506ppm | 552ppm |
| XRD | CHA | CHA | CHA | CHA | CHA | CHA | CHA |

## Claims

1. A boron-containing molecular sieve having the CHA crystal structure and comprising (1) silicon oxide and (2) a combination of boron oxide and aluminum oxide, and optionally iron oxide, titanium oxide, gallium oxide and mixtures thereof, wherein oxide (2) is more than 50% boron oxide on a molar basis.

2. A method for preparing a boron-containing molecular sieve according to claim 1, said method comprising:
A. forming an aqueous reaction mixture consisting of a composition in terms of mole ratios falling within the following ranges:
| | |
|---|---|
| YO₂/WₐO_{b} | >2 - 2,000 |
| OH⁻/YO₂ | 0.2 - 0.45 |
| Q/YO₂ | 0.2 - 0.45 |
| M_{2/n}O/YO₂ | 0 - 0.25 |
| H₂O/YO₂ | 22 - 80 |
wherein Y is silicon; W is a combination of boron and aluminum, and optionally iron, titanium, gallium and mixtures thereof; a is 1 or 2 and b is 2 when a is 1 or b is 3 when a is 2; M is an alkali metal or alkaline earth metal, n is the valence of M, and Q is a quaternary ammonium cation derived from 1-adamantamine, 3-quinuclidinol or 2-exo-aminonorbornane; and wherein Q is associated with an anion, A⁻, which is not detrimental to the formation of the molecular sieve, and wherein A⁻ is hydroxide; and
B. maintaining said aqueous mixture under sufficient crystallization conditions until crystals are formed.

3. A boron-containing molecular sieve according to claim 1 having a composition, as-synthesized and in the anhydrous state, in terms of mole ratios of oxides as follows:
| | |
|---|---|
| YO₂/W_{c}O_{d} | 20 - 2,000 |
| M_{2/n}O/YO₂ | 0 - 0.03 |
| Q/YO₂ | 0.02 - 0.05 |
wherein Y is silicon, W is a combination of boron and aluminum, and optionally iron, titanium, gallium and mixtures thereof; c is 1 or 2; d is 2 when c is 1 or d is 3 or 5 when c is 2; M is an alkali metal cation, alkaline earth metal cation or mixtures thereof; n is the valence of M (i.e., 1 or 2); and Q is a quaternary ammonium cation derived from 1-adamantamine, 3- quinuclidinol or 2-exo-aminonorbornane.

4. A process for the production of light olefins from a feedstock comprising an oxygenate or mixture of oxygenates, the process comprising reacting the feedstock at effective conditions over a catalyst comprising boron-containing molecular sieve as claimed in claim 1.

5. The process of claim 4 wherein the light olefins are ethylene, propylene, butylene or mixtures thereof.

6. The process of claim 5 wherein the light olefin is ethylene.

7. The process of claim 4 wherein the oxygenate is methanol, dimethyl ether or a mixture thereof.

8. The process of claim 7 wherein the oxygenate is methanol.

9. A process for producing methylamine or dimethylamine comprising reacting methanol, dimethyl ether or a mixture thereof and ammonia in the gaseous phase in the presence of a catalyst comprising a boron-containing molecular sieve as claimed in claim 1.

10. The process of claim 9 conducted at a temperature of from 250°C to 450°C.

11. A process for the reduction of oxides of nitrogen contained in a gas stream wherein said process comprises contacting the gas stream with the molecular sieve as claimed in claim 1.

12. The process of claim 11 conducted in the presence of oxygen.

13. The process of Claim 11 wherein said molecular sieve contains a metal or metal ions capable of catalyzing the reduction of the oxides of nitrogen.

14. The process of Claim 13 wherein the metal is cobalt, copper, platinum, iron, chromium, manganese, nickel, zinc, lanthanum, palladium, rhodium or mixtures thereof.

15. The process of Claim 11 wherein the gas stream is the exhaust stream of an internal combustion engine.

16. The process of Claim 14 wherein the gas stream is the exhaust stream of an internal combustion engine.

17. A process for separating gasses using a membrane containing a molecular sieve, the process comprising using as the molecular sieve a boron-containing molecular sieve as claimed in claim 1.

18. A process for treating a cold-start engine exhaust gas stream containing hydrocarbons and other pollutants consisting of flowing said engine exhaust gas stream over a molecular sieve bed which preferentially adsorbs the hydrocarbons over water to provide a first exhaust stream, and flowing the first exhaust gas stream over a catalyst to convert any residual hydrocarbons and other pollutants contained in the first exhaust gas stream to innocuous products and provide a treated exhaust stream and discharging the treated exhaust stream into the atmosphere, the molecular sieve bed **characterized in that** it comprises a boron-containing molecular sieve as claimed in claim 1.

19. The process of claim 18 wherein the molecular sieve has a mole ratio of oxide (1) to oxide (2) of 200-1500.

20. The process of claim 18 wherein the engine is an internal combustion engine.

21. The process of claim 20 wherein the internal combustion engine is an automobile engine.

22. The process of claim 18 wherein the engine is fueled by a hydrocarbonaceous fuel.

23. The process of claim 18 wherein the molecular sieve has deposited on it a metal selected from the group consisting of platinum, palladium, rhodium, ruthenium, and mixtures thereof.

24. The process of claim 23 wherein the metal is platinum.

25. The process of claim 23 wherein the metal is palladium.

26. The process of claim 23 wherein the metal is a mixture of platinum and palladium.

## Patentansprüche

1. Borhaltiges Molekularsieb mit der CHA Kristallstruktur und umfassend (1) Silliziumoxid und (2) eine Kombination aus Boroxid und Aluminiumoxid, und wahlweise Eisenoxid, Titanoxid, Galliumoxid und deren Mischungen, worin Oxid (2) zu mehr als 50% aus Boroxid auf einer molaren Basis besteht.

2. Verfahren für die Herstellung eines borhaltigen Molekularsiebs gemäß Anspruch 1, das Verfahren umfassend:
A. Bilden einer wässrigen Reaktionsmischung, bestehend aus einer Zusammensetzung, welche, in Bezug auf Molverhältnisse, in die folgenden Bereiche fällt:
| | |
|---|---|
| YO₂ / WₐO_{b} | > 2 bis 2000 |
| OH⁻ / YO₂ | 0,2 bis 0,45 |
| Q / YO₂ | 0,2 bis 0,45 |
| M_{2/n}O / YO₂ | 0 bis 0,25 |
| H₂O / YO₂ | 22 bis 80 |
worin Y Silizium ist; W eine Kombination aus Bor und Aluminium ist, und wahlweise Eisen, Titan, Gallium und deren Mischungen; a 1 oder 2 und b 2 ist, wenn a 1 ist oder b 3 ist, wenn a 2 ist; M ein Alkalimetall oder Erdalkalimetall ist, n die Wertigkeit von M ist, und Q ein quaternäres Ammoniumkation ist, abgeleitet von 1-Adamantamin, 3-Chinuclidinol oder 2-exo-Aminonorbornan; und worin Q mit einem Anion, A⁻, assoziert ist, welches für die Bildung des Molekularsiebs nicht schädlich ist, und worin A⁻ ein Hydroxid ist; und
B. Beibehalten der wässrigen Mischung unter ausreichenden Kristallisationsbedingungen, bis sich Kristalle gebildet haben.

3. Borhaltiges Molekularsieb gemäß Anspruch 1 mit einer Zusammensetzung, wie synthetisiert und in der wasserfreien Form, in Bezug auf Molverhältnisse der Oxide, wie folgt:
| | |
|---|---|
| YO₂ / W_{c}O_{d} | 20 bis 2000 |
| M_{2/n}O / YO₂ | 0 bis 0,03 |
| Q / YO₂ | 0,02 bis 0,05 |
worin Y Silizium ist; W eine Kombination aus Bor und Aluminium, und wahlweise Eisen, Titan, Gallium und deren Mischungen ist; c 1 oder 2 ist; d 2 ist, wenn c 1 ist oder d 3 oder 5 ist, wenn c 2 ist; M ein Alkalimetallkation, Erdalkalimetallkation oder Mischungen davon ist; n die Wertigkeit von M ist (das heißt, 1 oder 2); und Q ein quaternäres Ammoniumkation ist, abgeleitet von 1-Adamantamin, 3-Chinuclidinol oder 2-exo-Aminonorbornan.

4. Verfahren für die Herstellung von leichten Olefinen aus einem Ausgangsmaterial, umfassend ein Oxygenat oder eine Mischung von Oxygenaten, wobei das Verfahren umfasst die Reaktion des Ausgangsmaterials bei effektiven Bedingungen über einem Katalysator, umfassend das borhaltige Molekularsieb aus Anspruch 1.

5. Verfahren gemäß Anspruch 4, worin die leichten Olefine Ethylen, Propylen, Butylen oder deren Mischungen sind.

6. Verfahren gemäß Anspruch 5, worin das leichte Olefin Ethylen ist.

7. Verfahren gemäß Anspruch 4, worin das Oxygenat Methanol, Dimethylether oder deren Mischung ist.

8. Verfahren gemäß Anspruch 7, worin das Oxygenat Methanol ist.

9. Verfahren für die Herstellung von Methylamin oder Dimethylamin, umfassend die Reaktion von Methanol, Dimethylether oder deren Mischung mit Ammoniak in der Gasphase, in der Anwesenheit eines Katalysators, umfassend ein borhaltiges Molekularsieb nach Anspruch 1.

10. Verfahren gemäß Anspruch 9, ausgeführt bei einer Temperatur von 250°C bis 450°C.

11. Verfahren für die Reduktion von Stickoxiden, enthalten in einem Gasstrom, worin das Verfahren Zusammenbringen des Gasstroms mit dem Molekularsieb aus Anspruch 1 umfasst.

12. Verfahren gemäß Anspruch 11, ausgeführt in der Anwesenheit von Sauerstoff.

13. Verfahren gemäß Anspruch 11, worin das Molekularsieb ein Metall oder Metallionen enthält, in der Lage, die Reduktion von Stickoxiden zu katalysieren.

14. Verfahren gemäß Anspruch 13, worin das Metall Kobalt, Kupfer, Platin, Eisen, Chrom, Mangan, Nickel, Zink, Lanthan, Palladium, Rhodium oder deren Mischungen ist.

15. Verfahren gemäß Anspruch 11, worin der Gasstrom der Abgasstrom eines Verbrennungsmotors ist.

16. Verfahren gemäß Anspruch 14, worin der Gasstrom der Abgasstrom eines Verbrennungsmotors ist.

17. Verfahren für die Trennung von Gasen, unter Verwendung einer ein Molekularsieb enthaltenden Membran, wobei das Verfahren die Verwendung eines borhaltigen Molekularsiebs nach Anspruch 1 als das Molekularsieb umfasst.

18. Verfahren für die Behandlung eines Kaltstart-Motorabgasstroms, der Kohlenwasserstoffe und andere Verunreinigungen enthält, bestehend aus dem Strömenlassen des Motorabgasstroms über ein Molekularsiebbett, welches vorzugsweise die Kohlenwasserstoffe über Wasser adsorbiert, um einen ersten Abgasstrom bereitzustellen, und dem Strömenlassen des ersten Abgasstroms über einen Katalysator, um jegliche Kohlenwasserstoffe und andere Verunreinigungen, die im ersten Abgasstrom enthalten sind, in unschädliche Produkte umzuwandeln, und einen behandelten Abgasstrom bereitzustellen, und Ausstossen des behandelten Abgasstroms in die Atmosphäre, wobei das Molekularsiebbett **dadurch gekennzeichnet ist, dass** es ein borhaltiges Molekularsieb nach Anspruch 1 umfasst.

19. Verfahren gemäß Anspruch 18, worin das Molekularsieb ein Molverhältnis von Oxid (1) zu Oxid (2) von 200 bis 1500 hat.

20. Verfahren gemäß Anspruch 18, worin der Motor ein Verbrennungsmotor ist.

21. Verfahren gemäß Anspruch 20, worin der Verbrennungsmotor ein Kraftfahrzeugmotor ist.

22. Verfahren gemäß Anspruch 18, worin der Motor mit einem kohlenwasserstoffhaltigen Kraftstoff betrieben wird.

23. Verfahren gemäß Anspruch 18, worin auf dem Molekularsieb ein Metall abgeschieden ist, ausgewählt aus der Gruppe Platin, Palladium, Rhodium, Ruthenium und deren Mischungen.

24. Verfahren gemäß Anspruch 23, worin das Metall Platin ist.

25. Verfahren gemäß Anspruch 23, worin das Metall Palladium ist.

26. Verfahren gemäß Anspruch 23, worin das Metall eine Mischung von Platin und Palladium ist.

## Revendications

1. Un tamis moléculaire contenant du bore ayant la structure cristalline CHA et comprenant (1) de l'oxyde de silicium et (2) une combinaison de l'oxyde de bore et de l'oxyde d'aluminium, et éventuellement de l'oxyde de fer, de l'oxyde de titane, de l'oxyde de gallium et de leurs mélanges, dans lequel l'oxyde (2) est plus de 50% d'oxyde de bore sur une base molaire.

2. Un procédé pour la préparation d'un tamis moléculaire contenant du bore selon la revendication 1, ledit procédé comprenant:
A. former un mélange réactionnel aqueux constitué en une composition, en termes de rapports molaires se situant dans les plages suivantes:
| | |
|---|---|
| YO₂ / WₐO_{b} | > 2 à 2000 |
| OH- / YO₂ | 0,2 à 0,45 |
| Q / YO₂ | 0,2 à 0,45 |
| M_{2/n}O / YO₂ | 0 à 0,25 |
| H₂O / YO₂ | 22 à 80 |
dans lequel Y est le silicium; W est une combinaison du bore et de l'aluminium, et éventuellement du fer, du titane, du gallium et de leurs mélanges; a est égal à 1 ou 2 et b est égal à 2, lorsque a est égal à 1 ou b est égal à 3, lorsque a est égal à 2; M est un métal alcalin ou un métal alcalino-terreux, n est la valence de M, et Q est un cation d'ammonium quaternaire, dérivé de la 1-adamantamine, du 3-quinuclidinol ou du 2-exo-amino-norbornane; et dans lequel Q est associé à un anion, A⁻, qui n'est pas nuisible pour la formation du tamis moléculaire, et dans lequel A⁻ est un hydroxyde; et
B. maintenir ledit mélange aqueux dans des conditions de cristallisation suffisantes jusqu'à la formation de cristaux.

3. Un tamis moléculaire contenant du bore selon la revendication 1 ayant une composition, comme synthétisée et dans l'état anhydre, en termes de rapports molaires des oxydes comme suite:
| | |
|---|---|
| YO₂ / W_{c}O_{d} | 20 à 2000 |
| M_{2/n}O / YO₂ | 0 à 0,03 |
| Q / YO₂ | 0,02 à 0,05 |
dans lequel Y est le silicium; W est une combinaison du bore et de l'aluminium, et éventuellement du fer, du titane, du gallium et de leurs mélanges; c est égal à 1 ou 2; d est égal à 2, lorsque c est égal à 1 ou d est égal à 3 ou 5, lorsque c est égal à 2; M est un cation de métal alcalin, un cation de métal alcalino-terreux ou leurs mélanges; n est la valence de M (c'est-à-dire 1 ou 2); et Q est un cation d'ammonium quaternaire, dérivé de la 1-adamantamine, du 3-quinuclidinol ou du 2-exo-aminonorbornane.

4. Un procédé pour la production d'oléfines légères à partir d'une charge d'alimentation comprenant un composé oxygéné ou un mélange de composés oxygénés, le procédé comprenant la réaction de la charge d'alimentation dans des conditions efficaces sur un catalyseur comprenant le tamis moléculaire contenant du bore selon la revendication 1.

5. Le procédé selon la revendication 4, dans lequel les oléfines légères sont l'éthylène, le propylène, le butylène ou leurs mélanges.

6. Le procédé selon la revendication 5, dans lequel l'oléfine légère est l'éthylène.

7. Le procédé selon la revendication 4, dans lequel le composé oxygéné est le méthanol, l'éther diméthylique, ou un de leurs mélanges.

8. Le procédé selon la revendication 7, dans lequel le composé oxygéné est le méthanol.

9. Un procédé pour la production de la méthylamine ou de la diméthylamine comprenant la réaction du méthanol, de l'éther diméthylique ou un de leurs mélanges et de l'ammoniac en phase gazeuse en présence d'un catalyseur comprenant un tamis moléculaire contenant du bore selon la revendication 1.

10. Le procédé selon la revendication 9, mené à une température de 250°C à 450°C.

11. Un procédé pour la réduction des oxydes d'azotes contenus dans un flux de gaz, dans lequel ledit procédé comprend la mise en contact du flux de gaz avec le tamis moléculaire selon la revendication 1.

12. Le procédé selon la revendication 11, mené en présence d'oxygène.

13. Le procédé selon la revendication 11, dans lequel ledit tamis moléculaire contient un métal ou des ions métalliques capables de catalyser la réduction des oxydes d'azote.

14. Le procédé selon la revendication 13, dans lequel le métal est le cobalt, le cuivre, le platine, le fer, le chrome, le manganèse, le nickel, le zinc, le lanthane, le palladium, le rhodium ou leurs mélanges.

15. Le procédé selon la revendication 11, dans lequel le flux de gaz est le flux d'échappement d'un moteur à combustion interne.

16. Le procédé selon la revendication 14, dans lequel le flux de gaz est le flux d'échappement d'un moteur à combustion interne.

17. Un procédé pour la séparation de gaz en utilisant une membrane contenant un tamis moléculaire, le procédé comprenant l'utilisation comme le tamis moléculaire d'un tamis moléculaire contenant du bore selon la revendication 1.

18. Un procédé pour le traitement d'un flux de gaz d'échappement de moteur à démarrage froid contenant des hydrocarbures et d'autres polluants consistant à faire couler ledit flux de gaz d'échappement de moteur sur un lit de tamis moléculaire qui adsorbe préférentiellement les hydrocarbures sur l'eau pour fournir un premier flux d'échappement, et faire couler le premier flux de gaz d'échappement sur un catalyseur pour convertir tous les hydrocarbures résiduels et les autres polluants contenus dans le premier flux de gaz d'échappement en produits inoffensifs et fournir et fournir un flux d'échappement traité et décharger le flux d'échappement traité dans l'atmosphère, le lit de tamis moléculaire étant **caractérisé en ce qu'**il comprend un tamis moléculaire contenant du bore selon la revendication 1.

19. Le procédé selon la revendication 18, dans lequel le tamis moléculaire a un rapport molaire de l'oxyde (1) à l'oxyde (2) de 200 à 1500.

20. Le procédé selon la revendication 18, dans lequel le moteur est un moteur à combustion interne.

21. Le procédé selon la revendication 20, dans lequel le moteur à combustion interne est un moteur d'automobile.

22. Le procédé selon la revendication 18, dans lequel le moteur est alimenté par un carburant hydrocarboné.

23. Le procédé selon la revendication 18, dans lequel sur le tamis moléculaire est déposé un métal choisi parmi le groupe constitué en le platine, le palladium, le rhodium, le ruthénium, et leurs mélanges.

24. Le procédé selon la revendication 23, dans lequel le métal est le platine.

25. Le procédé selon la revendication 23, dans lequel le métal est le palladium.

26. Le procédé selon la revendication 23, dans lequel le métal est un mélange du platine et du palladium.
